# FASCICULE DE BREVET EUROPEEN

(11) **EP 2 593 129 B1**
(45) Date de publication et mention de la délivrance du brevet: **18.03.2015**
(21) Numéro de dépôt: 11752254.0
(22) Date de dépôt: 11.07.2011
(51) Int. Cl.: A61K 38/32, A61P 25/28, A61K 38/08

(54) **NONAPEPTIDE PAT POUR SON UTILISATION DANS LE TRAITEMENT DE LA MALADIE D'ALZHEIMER**
PAT-NONAPEPTIDS ZUR VERWENDUNG ZUR BEHANDLUNG VON ALZHEIMER ERKRANKUNGEN
PAT NONAPEPTIDE FOR USE IN TREATING ALZHEIMER'S DISEASE

(30) Priorité: 12.07.2010 FR 1002965
(43) Date de publication de la demande: 22.05.2013
(73) Titulaire: CLL Pharma, 06299 Nice cedex 3 (FR)
(72) Inventeur: TEMSAMANI, Jamal, F-30900 Nîmes (FR); LARUELLE, Claude, F-06270 Villeneuve-Loubet (FR)
(74) Mandataire: Peaucelle, Chantal
(86) Numéro de dépôt international: PCT/FR2011/000410
(87) Numéro de publication internationale: WO 2012/007658

(56) Documents cités:
- EP-A2- 0 425 828
- FR-A1- 2 830 451
- FR-A1- 2 930 156
- US-A- 5 112 810
- SAFIEH-GARABEDIAN B ET AL: "Thymulin reverses inflammatory hyperalgesia and modulates the increased concentration of proinflammatory cytokines induced by i.c.v. endotoxin injection.", NEUROSCIENCE 2003 LNKD- PUBMED:14580936, vol. 121, no. 4, 2003, pages 865-873, XP002607243, ISSN: 0306-4522
- MOREL G R ET AL: "Peripheral and mesencephalic transfer of a synthetic gene for the thymic peptide thymulin", BRAIN RESEARCH BULLETIN, ELSEVIER SCIENCE LTD, OXFORD, GB LNKD- DOI:10.1016/J.BRAINRESBULL.2006.03.015, vol. 69, no. 6, 31 mai 2006 (2006-05-31), pages 647-651, XP024900705, ISSN: 0361-9230 [extrait le 2006-05-31]
- HADDAD J J ET AL: "Thymulin: An emerging anti-inflammatory molecule", CURRENT MEDICINAL CHEMISTRY - ANTI-INFLAMMATORY & ANTI-ALLERGY AGENTS, vol. 4, no. 3, juin 2005 (2005-06), pages 333-338, XP009140580, ISSN: 1568-0142
- LICASTRO F ET AL: "Zinc and thymic hormone-dependent immunity in normal ageing and in patients with senile dementia of the Alzheimer type", JOURNAL OF NEUROIMMUNOLOGY, ELSEVIER SCIENCE PUBLISHERS BV, XX LNKD- DOI:10.1016/0165-5728(90)90070-4, vol. 27, no. 2-3, 1 mai 1990 (1990-05-01), pages 201-208, XP024016432, ISSN: 0165-5728 [extrait le 1990-05-01]
- DAVIS L J ET AL: "Plasmatic activity of thymulin: Comparison of free and protein bound thymulin in Alzheimer's disease", ADVANCES IN THE BIOSCIENCES; ALZHEIMER'S DISEASE AND RELATED DISORDERS PERGAMON PRESS LTD, HEADINGTON HILL HALL, OXFORD OX3 0BW, ENGLAND; PERGAMON PRESS INC., MAXWELL HOUSE, FAIRVIEW PARK, TARRYTOWN, NEW YORK 10523, USA SERIES : ADVANCES IN THE BIOSC, 1993, pages 279-280, XP009140532, & IIIRD INTERNATIONAL CONFERENCE; PADOVA, ITALY; JULY 12-17, 1993

## Description

L'invention concerne le composé constitué par le nonapeptide PAT répondant à la formule (I) :

EAKSQGGSD ;

ou un de ses sels pharmaceutiquement acceptables, pour son utilisation dans la prévention ou le traitement de la maladie d'Alzheimer.

Les maladies neurodégénératives affectent de façon progressive le fonctionnement du cerveau ou plus généralement le système nerveux. Le processus en cause consiste généralement en une détérioration du fonctionnement des cellules nerveuses, en particulier des neurones, voire en leur mort cellulaire. La conséquence pour le malade est donc une altération progressive souvent irréversible des fonctions nerveuses qui peut provoquer son décès. Le tableau clinique peut être soit une atteinte des fonctions psychiques, aboutissant à la démence comme dans la maladie d'Alzheimer ou la maladie de Pick, soit des anomalies motrices comme dans la sclérose latérale amyotrophique ou la maladie de Parkinson, soit l'association des deux comme dans la chorée de Huntington ou la maladie de Creutzfeldt-Jakob.

La maladie d'Alzheimer (MA) est la plus connue et la plus répandue des maladies neurodégénératives. Elle est caractérisée par une perte de mémoire et quelquefois par des troubles du raisonnement, de l'organisation, du langage et de la perception. Il est maintenant admis que les symptômes de MA proviennent d'une augmentation de la production ou de l'accumulation d'une protéine spécifique (β-amyloïde) dans le cerveau qui entraîne la mort de la cellule nerveuse. Le risque de survenue de la maladie d'Alzheimer augmente de manière exponentielle avec l'âge. Environ 30 millions de personnes dans le monde sont atteintes par la MA. Le vieillissement de la population fait penser que le poids économique occasionné par la MA deviendra de plus en plus important.

Bien qu'il n'y ait jusqu'à présent aucun traitement permettant la guérison de la MA, il existe 2 types de médicaments qui diminuent ses symptômes et ralentissent son évolution. EP-236684 A, DE 3805744 A et EP 296560 A divulguent des médicaments à base d'inhibiteurs d'acétylcholinestérase : galantamine, rivastigmine et donépézil. EP 392059 A divulgue un médicament à base de mémantine qui est un antagoniste du récepteur NMDA. Tous ces médicaments ont reçu l'autorisation de mise sur le marché pour traiter la MA. Cependant, leurs effets ne traitent que les symptômes. Plusieurs études ont montré que tous permettent seulement de ralentir de façon modeste la progression des symptômes cognitifs ainsi que les comportements problématiques chez certaines personnes. D'autre part, la moitié des patients ayant pris ces médicaments ne répondent pas aux traitements. Enfin, ces médicaments entraînent plusieurs effets indésirables tels nausées, diarrhées, troubles hépatiques etc... Il existe donc un besoin urgent de médicaments ayant un mécanisme d'action différent de ceux listés auparavant. Plusieurs pistes sont explorées à l'heure actuelle. Nous citerons ci-après quelques exemples.

Les inhibiteurs de la sécrétase bloquent le processus de transformation du précurseur de la protéine β (dit « APP ») en protéine amyloïde β et permettent de freiner l'accumulation dangereuse de cette dernière dans le cerveau. Parmi ces inhibiteurs, on trouve le tramiprosate (Alzhemed®), testé en clinique au cours d'une phase II (Aisen PS, Saumier D, Briand R et al., Neurology. 2006 Nov 28;67(10):1757-63. Un autre inhibiteur, le scillo-cyclohexanehexol, a été testé chez l'animal avec succès (Mac Larin JA et al ; Nature Medicine 12, 801 - 808 (2006). Ces molécules interagissent avec les protéines amyloïdes β durant leur formation pour les empêcher de s'agglomérer et de constituer de petits agrégats qui détruisent les cellules nerveuses en se déposant en plaques solides. Cependant, ces agrégats ont déjà causé des dommages importants en se formant.

D'autres traitements tel l'ubiquitine (composé naturellement produit dans le cerveau) favorisent la disparition de la protéine amyloïde β avant que son taux d'accumulation dans le cerveau ne soit trop élevé (Taddei et al; Neuroscience letters ; 1993, vol. 151, n°2, pp. 158-161). Cependant les taux d'ubiquitine restent insuffisants chez les patients atteints de la maladie d'Alzheimer.

Une autre approche intéressante est la voie immunologique. WO 94/06476 A divulgue un nouveau type de médicament ayant une cible différente des molécules citées précédemment : l'Etanercept (ENBREL®) -protéine de fusion dirigée contre la cytokine pro-inflammatoire TNF-α (*tumor necrosis factor*). Une étude pilote récente réalisée sur une période de 6 mois a montré des résultats encourageants pour l'amélioration cognitive (Tobinick E; CNS Drugs ; 2009 ; 23 :713-25). Outre le fait que le projet se situe à une phase préliminaire au niveau clinique, l'administration du produit ENBREL® a été réalisée par voie péri-spinale afin de contourner son incapacité à passer dans le cerveau en traversant la barrière hémato-encéphalique (BHE) (Griffin S ; Journal of Neuroinflammation ; 2008 ; 5 :3). Mais ce type d'administration est lourd et douloureux pour le patient et demande un certain nombre de précautions : il doit être réalisé en milieu hospitalier. La présence de la BHE limite fortement le passage des molécules du plasma vers l'espace extracellulaire cérébral : très peu de médicaments conçus en laboratoire, dont l'ENBREL®, traversent cette barrière pour traiter les affections du cerveau.

Il existe donc un besoin de médicaments qui soient d'une part suffisamment efficaces pour traiter la MA, et qui d'autre part s'affranchissent d'une administration intracérébrale.

La Demanderesse se donne comme objectif la mise au point d'un médicament qui permettra de traiter la maladie d'Alzheimer sans présenter les inconvénients liés à l'utilisation des traitements existants tels que présentés précédemment. Elle a trouvé de façon fortuite, grâce aux travaux déjà effectués sur cette molécule, qu'un peptide analogue de l'hormone thymuline présente un potentiel intéressant dans la prévention et le traitement de la MA.

On connait depuis la fin des années 1950 le rôle central joué par le thymus dans la différenciation des lymphocytes T, responsables notamment du rejet des greffes et impliqués dans la défense immunitaire contre les virus et certaines bactéries. L'hormone, secrétée par le thymus fut alors identifiée comme un peptide de 9 acides aminés : la thymuline (Bach et al Pleau et al. Immunol letters, 1979; 1:179-182; Amor et al., Annals of the Rheumatic Diseas 1987 :46 :549-554). Les effets de la thymuline sur le système immunitaire dépendent de la présence du zinc. En effet, le zinc associé au peptide confère à celui-ci une conformation tétrahedrique qui correspond à la forme active de la molécule. En l'absence de zinc, la thymuline n'est plus active vis-à-vis du système immunitaire. Des travaux ont été entrepris spécifiquement sur un nonapeptide dénommé PA ou « *Peptide Analog of Thymulin*» ayant la séquence d'acides aminés Glu-Ala-Lys-Ser-Gln-Gly-Gly-Ser-Asp (EAKSQGGSD). La demande WO 03/030927A rapporte que des dérivés de la thymuline, tel le nonapeptide PAT, présentent des propriétés analgésiques et anti-inflammatoires, et peuvent traiter en particulier des douleurs d'origine neurogène périphérique. La demande WO 2009/150310A, plus récente, décrit de façon spécifique l'utilisation du nonapeptide PAT dans le traitement de maladies autoimmunes telles la polyarthrite rhumatoïde, ou celles appartenant à la catégorie des maladies inflammatoires chroniques intestinales (MICI) dont la maladie de Crohn et la rectocolite hémorragique font parti.

La présente invention se rapporte au composé constitué par le nonapeptide PAT répondant à la formule (I) :

EAKSQGGSD ;

ou un de ses sels pharmaceutiquement acceptables, pour son utilisation dans la prévention ou le traitement de la maladie d'Alzheimer.

Le peptide PAT est administré chez l'homme ou chez l'animal à un dosage compris entre 0,1 et 50 mg ; de préférence entre 1 et 10 mg.

Par "sel pharmaceutiquement acceptable", on entend de manière non limitative un acétate, un sulfate ou un chlorhydrate.

L'utilisation d'un composé de formule (I) dans laquelle un ou plusieurs acides aminés sont en configuration D est également décrite.

La composition pharmaceutique peut être administrée par voie parentérale, topique, orale, péri-linguale, rectale ou intraoculaire. La voie d'administration préférée est la voie parentérale, en particulier sous cutanée (s.c.), intranasale, intrapéritonéale (i.p.) et intraveineuse (i.v). On pourra également envisager de l'administrer sous une forme topique, notamment transdermique, par exemple sous la forme de patch, de pommade ou de gel.

Une expérimentation réalisée dans le cadre de l'invention sur un modèle animal d'Alzheimer a démontré que le nonapeptide PAT exerce une activité biologique lorsqu'il est administré par voie i.c.v. -et donc s'affranchit du passage à travers la BHE-, mais il est également actif lorsqu'il est administré par voie parentérale (intra-péritonéale dans les tests réalisés). Ce dernier point suggère que le produit passe dans le cerveau en traversant la BHE. Pour passer la BHE, l'homme du métier sait que les propriétés moléculaires et physicochimiques de la molécule doivent répondre aux 5 critères décrits par Lipinski et al. (1997). Adv Drug Del Rev 23: 3-25 (dont le poids moléculaire faible, la lipophilicité, la charge, etc...). Or, on s'étonne de constater que le peptide PAT, qui ne répond pas favorablement à tous ces critères, traverse la BHE.

Les formulations à administrer par voie parentérale contiennent un solvant convenant à la solubilisation d'un peptide tel le peptide PAT ; ce solvant peut être choisi parmi l'eau pour préparation injectable (ppi) ou le sérum physiologique, éventuellement accompagné de conservateurs (tels le crésol, le phénol, l'alcool benzylique ou le méthylparaben) et/ou d'agents tampons, et/ou d'adjuvant(s) d'isotonicité et/ou d'agent(s) tensioactif(s) bien connus par l'homme de l'art.

Une des voies d'administration préférée est la voie sous-cutanée (s.c.). La formulation injectable par voie sous-cutanée contient le peptide PAT dissout dans un solvant approprié, accompagné le cas échéant par d'autres excipients tels que ceux cités précédemment. Une des formulations injectables envisagée en sous-cutané est à base de polymères permettant une diffusion lente du peptide PAT au cours du temps (période pouvant atteindre 30 à 40 jours). Pour l'obtenir, on dissout préalablement le peptide PAT dans un solvant adapté tel le sérum physiologique, puis on ajoute à ce mélange des polymères tels des polyéthylèneglycols, des polyvinylpyrrolidones et/ou des polyacrylamides.

D'autres avantages et caractéristiques de l'invention apparaîtront à la lecture des exemples qui suivent. On fera référence aux dessins en annexe dans lesquels :
- la figure 1 montre les résultats obtenus au cours du test du labyrinthe en Y chez des souris ayant reçu préalablement par voie intracérébroventriculaire (i.c.v) le peptide Scaβ (« *scrambled* ») ou le peptide Aβ₂₅₋₃₅. et également par voie i.c.v un véhicule inerte (V) ou le peptide PAT. Le paramètre évalué est l'alternance spontanée exprimé en pourcentage ;
- la figure 2 montre les résultats obtenus lors du test d'évitement passif;
   les groupes traités sont identiques à ceux de la figure 1 ; dans la figure 2A on mesure le temps de latence (en sec) pour entrer dans le compartiment sombre ; et dans la figure 2B on mesure le temps de latence de fuite (en sec) ;
- la figure 3 montre les résultats obtenus au cours du test du labyrinthe en Y dans les mêmes conditions que pour la figure 1, mais le peptide PAT et le véhicule inerte (V) sont injectés par voie intra-péritonéale (i.p.) ;
- la figure 4 montre les résultats obtenus lors du test d'évitement passif tel celui réalisé dans la figure 2 mais le peptide PAT et le véhicule inerte (V) sont injectés par voie intra-péritonéale (i.p.) ; dans la figure 4A est mesuré le temps de latence (en sec) pour entrer dans le compartiment sombre ; et dans la figure 4B on mesure le temps de latence de fuite (en sec).

Dans les figures présentées ci-dessus, le test de Dunnett est appliqué aux résultats : (*) signifie que les résultats sont significatifs avec une probabilité p < 0.0001 ; (**) avec une *p* < 0.01 *vs.* Groupe de traitement ScAβ+V ; (#) avec une p < 0.05 *vs.* groupe de traitement (Aβ₂₅₋₃₅+V) ; et (##) avec une p < 0.01 *vs*. groupe de traitement (AP₂₅₋₃₅+V).

### EXEMPLE 1: MODELE D'ALZHEIMER CHEZ LA SOURIS-TEST D'ALTERNANCE SPONTANEE

### Protocole expérimental

Les souris Swiss OF-1 (Depré, St Doulchard, France) sont âgées de 7 à 9 semaines et pèsent 32±2 g. Elles sont réparties en plusieurs groupes dans des cages en plastique. Elles ont libre accès à la nourriture et à l'eau, sauf pendant l'expérimentation comportementale, et sont maintenues dans un environnement régulé (23±1°C, 40-60% d'humidité) avec des cycles lumière/obscurité de 12h (lumière à 8h du matin). Les expériences sont réalisées entre 9h du matin et 5h de l'après midi, dans une chambre d'expérimentation. Les souris sont mises en condition pendant 30 minutes avant le début de l'expérience. Chaque protocole est suivi en respectant strictement les directives de l'Union Européenne du 24 novembre 1986.

### Traitement

Le peptide PAT synthétisé par Polypeptide (Danemark), (5 µg) a été solubilisé dans de l'eau distillée et administré par voie intra-péritonéale (i.p.) dans un volume de 100 µl par 20 g de poids corporel ou par voie i.c.v. en même temps que le peptide amyloïde. Le peptide β amyloïde [25-35] appelé Aβ₂₅₋₃₅ et le peptide « scrambled » Aβ₂₅₋₃₅-dénommé Sc.Aβ- proviennent de Genepep (France). Ils sont dissous dans de l'eau distillée stérile à une concentration de 3 mg/ml et conservés à 20°C jusque leur utilisation. Avant leur administration, les peptides sont soumis à une agrégation à 37°C pendant 4 jours. Ils sont administrés par voie i.c.v. dans un volume final de 3µl par souris. Les animaux sont testés au Jour 7 après l'injection.

Dans une 1^{ière} série d'expérience, on administre aux souris par voie i.c.v. soit de l'eau, soit le nonapeptide PAT (5 µg) en même temps que le peptide ScAβ ou le peptide Aβ₂₅₋₃₅ (9 nmol). Après une période de 7 jours, on évalue chez les souris leur performance à l'alternance spontanée. Le nombre d'animaux par lot était respectivement de 10 et de 11. Dans une 2^{nde} série d'expérience, on réalise le même test que précédemment, mais le PAT est administré par voie intra-péritonéale i.p.

### Déroulement du test- Paramètres mesurés

On place chaque souris, qui ne connait pas l'appareil, à l'extrémité d'un bras d'un labyrinthe en Y (3 bras de 50 cm de long et séparés de 60°) et on la laisse se déplacer librement pendant 8 minutes. Le nombre d'entrées dans chaque bras, y compris les retours possibles sur le même bras, est compté visuellement. Une entrée est comptabilisée lorsque les pattes de devant de l'animal franchissent au moins 2 cm à l'intérieur du bras. Une alternance est comptabilisée lorsqu'une entrée est effectuée dans les 3 bras lors d'essais successifs. Le nombre d'alternances possibles totales est alors le nombre total d'entrées moins 2 et le pourcentage d'alternance est calculé ainsi: (alternances comptabilisées / total des alternances possible) X 100. Les animaux réalisant moins de 8 entrées en 8 minutes sont écartés des groupes expérimentaux. Aucun animal n'a été exclu dans cette étude. Les composés sont administrés 30 minutes avant la session.

### Résultats

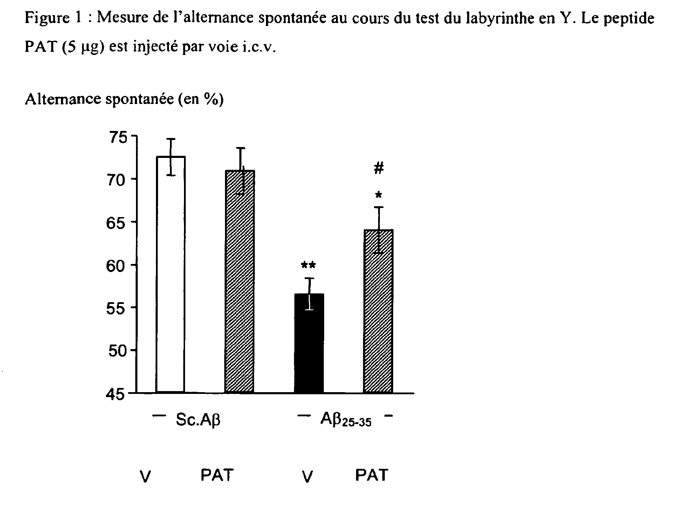

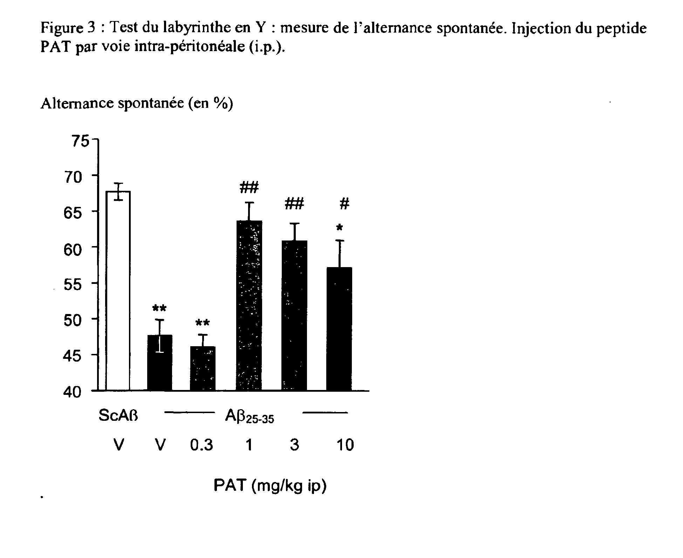

Les résultats sont montrés dans la figure 1 et la figure 3 pour les voies d'administration respectivement i.c.v. et i.p.

L'administration du peptide Aβ₂₅₋₃₅, comme prévu, a induit les symptômes de la maladie d'Alzheimer. En revanche, l'administration du peptide contrôle ScAβ n'a eu aucun effet.

De façon prévisible, on constate dans la figure 1 (voie i.c.v.) que lorsque le peptide ScAβ est administré aux souris (celles-ci ne reproduisant pas les symptômes de la maladie d'Alzheimer), la co-administration du peptide PAT n'a aucun effet. En revanche, lorsque le peptide Aβ₂₅₋₃₅ est administré (les souris reproduisant les symptômes de la MA au niveau de la mémoire), alors la co-administration du peptide PAT exerce à la dose de 5 µg un effet neuroprotecteur significatif sur les déficits d'apprentissage induits par le peptide Aβ₂₅₋₃₅.

Dans la figure 3 (voie i.p.), on constate également un effet neuroprotecteur du PAT et on note que cet effet est très significatif pour une dose de peptide PAT de 1 et 3 mg/kg.

### EXEMPLE 2: MODELE D'ALZHEIMER CHEZ LA SOURIS - TEST D'EVITEMENT PASSIF

### Protocole expérimental :

Les informations relatives aux souris, aux peptides, leur administration ainsi qu'aux groupes traités sont identiques à celles de l'Exemple 1.

### Déroulement du test- Paramètres testés

Les composés sont administrés 30 minutes avant l'exercice. Ce test permet d'évaluer la mémoire non spatiale à long terme. L'appareil du test consiste en un compartiment illuminé aux parois en PVC blanc (15 cm de largeur ; 20 cm de longueur ; et 15 cm de hauteur) ; un compartiment obscur aux parois en PVC noir (de mêmes dimensions) et une grille au sol. Une trappe sépare les 2 compartiments. Une lampe de 60W est placée 40 cm au dessus et éclaire le compartiment blanc durant l'expérience. Au niveau de la grille, les chocs électriques aléatoires de 0,3 mA sont délivrés aux pattes des souris pendant 3 secondes grâce à un générateur électrique aléatoire (Lafayette Instruments, USA).

La 1^{ière} phase de l'expérience, appelée apprentissage ou entraînement, a lieu. La trappe est fermée au début de l'exercice. Chaque souris est placée dans le compartiment blanc. La trappe est levée après 5 secondes. Quand la souris entre dans le compartiment sombre et appuie toutes ses pattes sur la grille, la trappe est fermée et le choc électrique aléatoire est délivré aux pattes pendant 3 secondes. Le temps de latence avant l'entrée dans le compartiment sombre et le nombre de réponses sont enregistrés. Le nombre de réponses ne diffère pas entre les groupes, indiquant que la sensibilité au choc électrique n'est pas affectée par le type de voie d'administration c'est-à-dire ici i.c.v ou i.p (résultats non montrés). Les animaux pour lesquels le temps de latence est en dehors de la fourchette de 3-30 secondes sont écartés de l'expérience. L'attrition compte pour moins de 2% des animaux et est indépendante du traitement.

La 2^{ième} phase de l'expérience, appelée rétention, est effectué 24 h après la 1^{ière} phase (entraînement). Chaque souris est placée de nouveau dans le compartiment blanc. La trappe est levée après 5 secondes. Le temps de latence d'entrée dans le compartiment sombre est enregistré pendant une durée de 300 secondes. Le nombre d'entrées et le temps de fuite (temps passé à retourner dans le compartiment blanc) sont mesurés pendant 300 secondes.

### Résultats

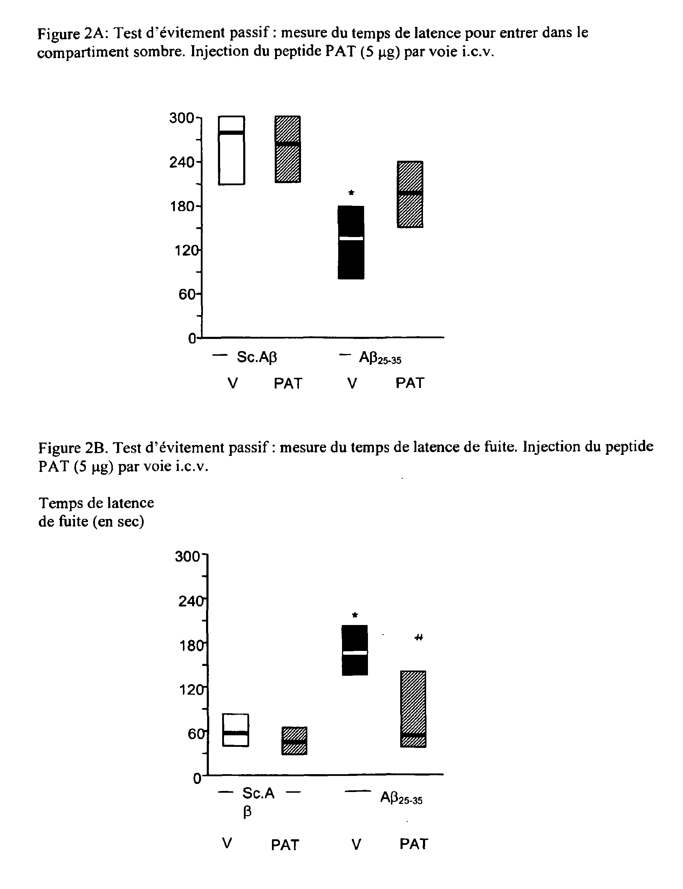

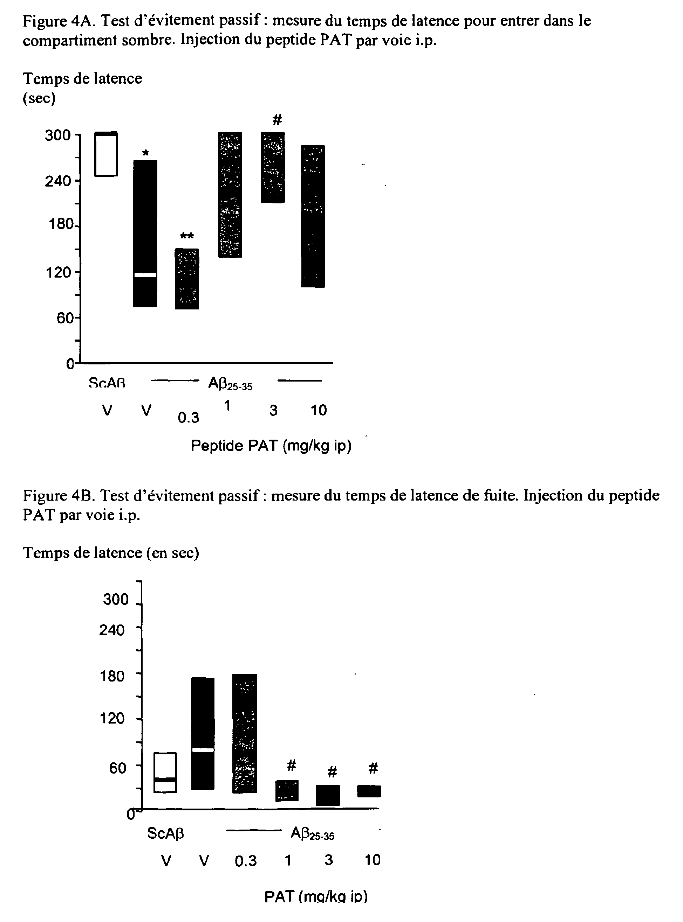

Les résultats sont présentés dans la figure 2 (2A et 2B) pour l'administration par voie i.c.v. ; et la figure 4 (4A et 4B) pour l'administration par voie i.p.

La figure 2 -administration par voie i.c.v- montre clairement que l'injection du PAT (5 µg) par voie i.p. à des souris ayant reçu le peptide Aβ₂₅₋₃₅ (ces derniers reproduisant les déficits d'apprentissage) améliore les 2 critères testés lorsqu'on les compare aux souris ayant reçu l'eau distillée seule (V). Ainsi, en utilisant ce modèle animal de la maladie d'Alzheimer, il est démontré que le peptide PAT présente de façon significative un effet neuroprotecteur.

De même, dans la figure 4 -administration par voie i.p.-, on observe un effet neuroprotecteur du PAT. Tout comme lors du test d'alternance spontanée, l'effet neuroprotecteur du PAT est visible pour une dose supérieure à 0,3 mg/kg de poids corporel.

Ainsi, l'utilisation des 2 modèles animaux de la maladie d'Alzheimer permet de démontrer que le peptide PAT est un candidat prometteur pour prémunir et traiter des lésions cérébrales liées au déficit de l'apprentissage.

## Revendications

1. Composé constitué par le nonapeptide PAT répondant à la formule (I) :
EAKSQGGSD ;
ou un de ses sels pharmaceutiquement acceptables, pour son utilisation dans la prévention ou le traitement de la maladie d'Alzheimer.

2. Composé pour son utilisation selon la revendication 1, **caractérisé en ce qu'**il est administré par voie parentérale.

3. Composé pour son utilisation selon la revendication 2, **caractérisé en ce que** la voie d'administration est sous-cutanée, intra-péritonéale, intraveineuse ou intranasale.

4. Composé pour son utilisation selon l'une quelconque des revendications 1 à 3, **caractérisé en ce que** le dosage est compris entre 0,1 et 50 mg.

5. Composé pour son utilisation selon la revendication 4, **caractérisé en ce que** le dosage est compris entre 1 et 10 mg.

6. Composé pour son utilisation selon l'une quelconque des revendications 1 à 5, **caractérisé en ce qu'**un ou plusieurs acides aminés du peptide se présente en configuration D.

7. Composé pour son utilisation selon l'une quelconque des revendications 1 à 6, **caractérisé en ce qu'**il se présente sous forme administrable par voie parentérale à des patients atteints par la maladie d'Alzheimer dissous dans un solvant approprié, éventuellement accompagné de conservateurs, agents tampons, adjuvants d'isotonicité et/ou agents tensioactifs.

## Patentansprüche

1. Verbindung, bestehend aus dem Nonapeptid PAT, das der Formel (I) entspricht:
EAKSQGGSD;
oder einem seiner pharmazeutisch akzeptablen Salze, für ihre Verwendung bei der Prävention oder der Behandlung der Alzheimer-Krankheit.

2. Verbindung für ihre Verwendung nach Anspruch 1, **dadurch gekennzeichnet, dass** sie auf parenteralem Weg verabreicht wird.

3. Verbindung für ihre Verwendung nach Anspruch 2, **dadurch gekennzeichnet, dass** der Verabreichungsweg subkutan, intraperitoneal, intravenös oder intranasal ist.

4. Verbindung für ihre Verwendung nach einem der Ansprüche 1 bis 3, **dadurch gekennzeichnet, dass** die Dosierung zwischen 0,1 und 50 mg beträgt.

5. Verbindung für ihre Verwendung nach Anspruch 4, **dadurch gekennzeichnet, dass** die Dosierung zwischen 1 und 10 mg beträgt.

6. Verbindung für ihre Verwendung nach einem der Ansprüche 1 bis 5, **dadurch gekennzeichnet, dass** eine oder mehrere Aminosäuren des Peptids in D-Konfiguration vorliegt/vorliegen.

7. Verbindung für ihre Verwendung nach einem der Ansprüche 1 bis 6, **dadurch gekennzeichnet, dass** sie in einer Form vorliegt, die Patienten, welche an der Alzheimer-Krankheit erkrankt sind, auf parenteralem Weg verabreichbar ist, in einem geeigneten Lösungsmittel gelöst ist, eventuell begleitet von Konservierungsmitteln, Puffermitteln, Isotonizitäts-Zusätzen und/oder Tensiden.

## Claims

1. Compound constituted by the PAT nonapeptide responding to the formula (I):
EAKSQGGSD;
or one of its pharmaceutically acceptable salts, for its use in the prevention or the treatment of Alzheimer's disease.

2. Compound for its use according to claim 1, **characterized in that** it is administered by parenteral route.

3. Compound for its use according to claim 2, **characterized in that** the route of administration is subcutaneous, intra-peritoneal, intravenous or intranasal.

4. Compound for its use according to anyone of claims 1-3, **characterized in that** the dose is ranged between 0.1 and 50 mg.

5. Compound for its use according to the claim 4, **characterized in that** the dose is ranged between 1 and 10 mg.

6. Compound for its use according to anyone of the claims 1-5, **characterized in that** at least one amino-acid of the peptide is under the D configuration.

7. Compound for its use according to anyone of the claims 1-6, **characterized in that** the PAT peptide is present under administrable form by parenteral route to patients suffering from Alzheimer's disease, dissolved in an appropriate solvent, optionally along with preservatives, buffering agents, isotonic adjuvants and/or tensioactive agents.


### SEQUENCE LISTING

<110> CLL PHARMA
<120> UTILISATION DU NANOPEPTIDE PAT DANS LE TRAITEMENT ET LA PREVENTION DES MALADIES NEURODEGENERATIVES
<130> CP/BB 63363-3936
<150> PCT/FR2011/000410
   <151> 2011-07-11
<150> FR 10/02965
   <151> 2010-07-12
<160> 1
<170> PatentIn version 3.5
<210> 1
   <211> 9
   <212> PRT
   <213> Artificial Sequence
<220>
   <223> Amino acids
<400> 1
   Glu Ala Lys Ser Gln Gly Gly Ser Asp
   1               5